# EUROPÄISCHE PATENTSCHRIFT

(11) **EP 0 590 321 B1**
(45) Veröffentlichungstag und Bekanntmachung des Hinweises auf die Patenterteilung: **14.01.1998**
(21) Anmeldenummer: 93113775.6
(22) Anmeldetag: 27.08.1993
(51) Int. Cl.: A61K 31/70

(54) **Verwendung von NADH und NADPH zur Herstellung eines Arzneimittels zur Behandlung von Morbus Alzheimer**
Use of NADH and NADPH for the manufacture of a medicament for the treatment of Alzheimer's disease
Utilisation de NADH et NADPH pour la fabrication d'un médicament pour le traitement de la maladie d'Alzheimer

(30) Priorität: 30.09.1992 DE 4232899
(43) Veröffentlichungstag der Anmeldung: 06.04.1994
(73) Patentinhaber: Birkmayer, Jörg, Univ.-Prof. DDr., A-1090 Wien (AT)
(72) Erfinder: Birkmayer, Jörg, Univ.-Prof. DDr., A-1090 Wien (AT)
(74) Vertreter: von Hellfeld, Axel, Dr. Dipl.-Phys.

(56) Entgegenhaltungen:
- EP-A- 0 345 247

## Beschreibung

Die Erfindung betrifft eine neuartige Verwendung der reduzierten Formen von Nikotinamid-adenin-dinukleotid (NADH) oder Nikotinamid-adenin-dinukleotidphosphat (NADPH) oder eines physiologisch verträglichen Salzes derselben.

Die Alzheimer-Krankheit (Morbus Alzheimer) kann auch als präsenile Demenz vom Alzheimer-Typ (DAT) oder in der modernen amerikanischen psychiatrischen Nomenklatur als primäre degenerative Demenz bezeichnet werden. Sie zeichnet sich durch folgende Symptome aus: Abnahme der kognitiven Fähigkeiten, Vergeßlichkeit, Konfusionen, Abnahme des Kurzzeit- und Langzeitgedächtnisses sowie der Orientierung und herabgesetzte Selbsterhaltungs- und Selbstbetreuungsfähigkeit (self-care-ability). Neuropathologisch finden sich im Gehirn von Alzheimer-Patienten Amyloid-Ablagerungen, degenerierte Neurite sowie proliferierende Glia-Zellen als Teile von senilen Plaques.

Biochemisch findet man eine Störung des Neurotransmitter-Stoffwechsels, insbesondere des Acetylcholins. Es kommt zu einem selektiven Verlust der cholinergen Neuronen. Neuerdings hat man Veränderungen in der Zellmembran gefunden, die den Phospholipid-Umsatz beeinflussen (Petecroof 1989, Annals New York-Academy of Science 586 C, Seite 5 - 28).

Zur Zeit gibt es keine Substanz, die den Gedächtnisschwund und die Abnahme anderer kognitiver Fähigkeiten bei Patienten mit Morbus Alheimer aufhebt, ein Fortschreiten verzögert oder der Entwicklung dieser Krankheit entgegenwirkt. Da für die kognitiven Fähigkeiten vor allem der Neurotransmitter Acetylcholin wichtig ist, hat man versucht, auf dessen Synthese bzw. Abbau einzuwirken. Als Cholinomimetika wurden Arencholin, Teanol und Oxotremorin an Tiermodellen untersucht, die Acetylcholinesterase-Hemmer Etrophonium und Tacrin wurden auch in klinischen Studien an Menschen getestet. Bei beiden Substanzen, insbesondere bei Tacrin, zeigte sich nach einer Doppelblindstudie, daß kein signifikanter Unterschied zwischen Tacrin und Placebo in Verbesserung der Symptomatik bei Alzheimer-Patienten zu beobachten war. Alle bisher zur Behandlung des M. Alzheimer ins Auge gefaßten Substanzen haben die Erwartung nach klinischer Prüfung nicht erfüllt.

Es besteht daher nach wie vor ein großes Bedürfnis nach einem Medikament zur wirksamen Behandlung von Morbus Alzheimer. Aufgabe der vorliegenden Erfindung ist es daher, ein derartiges Therapeutikum zur Verfügung zu stellen.

Die Erfindung betrifft die Verwendung der reduzierten Form von Nikotinamid-adenin-dinukleotid (NADH), der reduzierten Form von Nikotinamid-adenin-dinukleotidphosphat (NADPH) oder eines physiologisch verträglichen Salzes derselben zur Herstellung eines Arzneimittels (Medikament) zur Behandlung von Morbus Alzheimer. Es kommen Einzeldosen zwischen 1 und 50 mg, bevorzugt 1 bis 25 mg NADH und 1 bis 20 mg NAPD zum Einsatz.

Aus der EP-A-0 345 247 ist die gemeinschaftliche Verwendung von NADH bzw. NADPH mit monosubstituierten selenorganischen Verbindungen, wie beispielsweise Selenmethionin zur Behandlung des Parkinson-Syndroms, des Morbus Alzheimer oder anderer Formen präseniler Demenz bekannt. Die gemeinschaftliche Verwendung von NADH bzw. NADPH mit monosubstituierten selenorganischen Verbindungen zur Herstellung eines Arzneimittels zur Behandlung von Morbus Alzheimer wird folglich vom Schutz ausgenommen.

Die Einzeldosen werden entweder oral oder parenteral (beispielsweise intravenös) verabreicht. Die Verabreichung erfolgt täglich (gegebenenfalls mehrfach täglich) oder mit Tagesabständen zwischen den einzelnen Verabreichungen, wie beispielsweise 2 mal pro Woche (beispielsweise Tag 1 und Tag 4 der Woche) oder jeden zweiten bzw. dritten Tag. Bei oraler Gabe werden üblicherweise 2,5 bis 10 mg NADH und 1 bis 5 mg NADPH verabreicht. Bei parenteraler Verabreichung haben sich 1 bis 50 mg NADH und 1 bis 20 m g NADPH bewährt.

Der Einsatz dieser körpereigenen Substanzen führt zu überraschenden therapeutischen Erfolgen, ohne daß Nebenwirkungen zu erkennen oder zu erwarten sind. Bei Patienten mit seniler oder präseniler Demenz vom Alzheimer-Typ wurde nach Verabreichung von NADH bzw. NADPH eine deutliche Verbesserung ihrer kognitiven Funktion, ihres Gedächtnisses, ihrer Orientierungsfähigkeit und ihrer Fähigkeit zur Selbstbetreuung erreicht. Nach 3 bis 4 Wochen kam es zu einer signifikanten Verbesserung der intellektuellen Leistungsfähigkeit. Beim Abbruch der Therapie hält die Wirkung in der Regel etwa 4 bis 6 Wochen an. Eine Rückkehr zum ursprünglichen Ausgangszustand konnte aber in keinem Fall festgestellt werden.

### FALLBESCHREIBUNGEN

- Fall 1:: Patient, weiblich, 58 Jahre alt, Erstuntersuchung fand am 10.5.1989 statt. Die Patientin war seit einigen Jahren vergeßlich und der Zustand verschlechterte sich nach Angaben der Patientin laufend weiter. Die Patientin findet nach dem Einkaufen nicht mehr nach Hause, weiß auch nicht mehr, was sie einkaufen soll, findet sich in ihrer eigenen Wohnung nicht mehr zurecht, findet von einem Zimmer nicht in das andere und wünscht von ihrem Mann die Einlieferung in eine Krankenanstalt. Der Global-Deterioration-Score(GDS) beträgt 5, der Mini-Mental-State(MMS)-Score beträgt 8.
Diagnose: Morbus Alzheimer.

Therapie: NADH 5 mg täglich, Kontrolle am 12.6.1989, die kognitiven Fähigkeiten der Patientin haben sich auffällig verbessert, sie kann wieder einkaufen gehen, sie findet sich in der Wohnung zurecht, sie sieht fern und versteht die Nachrichten wieder. Objektiv nimmt der GDS-Wert auf 3 ab, der MMS-Wert auf 20 zu. Die Therapie mit NADH ist mit kurzen Unterbrechungen bis heute fortgesetzt worden. Der Zustand hat sich noch etwas gebessert. Es wurden keinerlei Nebenwirkungen beobachtet, auch von der Patientin keine angegeben.
- Fall 2:: Patient, männlich, 65 Jahre alt, beobachtete vor etwa sechs Jahren erstmals Symptome von Vergeßlichkeit und Mangel an Erkennungsfähigkeit. Am 10. Oktober 1989 kam der Patient erstmalig zu einer neurologischen Untersuchung. Das Ergebnis war eine deutlich eingeschränkte Hirnleistungsfunktion mit einem GDS-Wert von 5 und einem MMS-Wert von 10: Probleme bei der Ortsfindung, Wortwiederholungen, einfache Rechenaufgaben (1 x 1) können nicht gelöst werden, Lesen ist ebenfalls nicht möglich.
Diagnose: Morbus Alheimer.

Therapie: NADH 5 mg oral 1x am Tag, Kontrolle am 16.11.1989. Die kognitiven Funktionen haben sich deutlich gebessert, Rechenaufgaben können nun gelöst werden, keine Probleme bei der Wortfindung, der GDS-Wert wurde mit 3 ermittelt, der MMS-Wert ist auf 22 angestiegen. Die Therapie wurde acht Wochen fortgesetzt, die Score-Werte bleiben im wesentlichen unverändert, danach wurde die Therapie ein Jahr ausgesetzt. Nach Sistieren der Therapie kam es zu einer neuerlichen Verschlechterung der Hirnleistungsfähigkeit, ein GDS-Score von 4 und ein MMS-Wert von 16 wurden danach registriert, worauf eine neuerliche NADH-Therapie (5 mg oral täglich) begonnen wurde, die eine neuerliche Verbesserung der Hirnleistungsfunktion ergeben hat.
- Fall 3:: Patient, 55 Jahre, weiblich, Erstuntersuchung am 18.5.1990, bei der eine stark eingeschränkte Hirnleistungsfunktion festgestellt wird. Extreme Wortarmut, häufige Wortwiederholungen, Fixierung auf bestimmte Reiterationen, Lesen einfacher Sätze sehr schwierig, Verstehen unmöglich, einfachste Rechenaufgaben können nicht gelöst werden, Ziffern rückwärts zählen nicht möglich. GDS-Wert 6, MMS-Wert 10.
Diagnose: Morbus Alzheimer

Therapie: NADH 5 mg oral täglich, Kontrolle am 22.6.1990: Kognitive Funktionen deutlich gebessert, Kurz- und Langzeitgedächtnis deutlich erhöht, einfache Rechenaufgaben können gelöst werden, der Sinn von Sprichwörtern kann nachvollzogen werden. Der GDS-Wert sinkt auf 3 ab, der MMS-Wert steigt auf 22 an. Therapie mit NADH (5 mg oral jeden zweiten Tag) wurde fortgesetzt, der am Kontrolluntersuchungstermin festgestellte Zustand konnte aufrechterhalten werden. Nebenwirkungen wurden vom Patienten nicht beobachtet.
- Fall 4:: Patient, 62 Jahre, männlich, Untersuchung am 13.2.1991. Im Vordergrund stehen eine ausgeprägte Vergeßlichkeit, sowie eine auffällige Teilnahmslosigkeit. Die Untersuchung gibt eine stark eingeschränkte kognitive Funktionsfähigkeit, die zeitliche und räumliche Orientiertheit ist herabgesetzt. Der Patient kann kein Datum angeben, erinnert sich an keine aktuelle Nachricht, neurologisch sonst organisch ohne Besonderheiten.
Diagnose: Morbus Alzheimer

Therapie: NADPH 5 mg intravenös, 2x die Woche, Kontrolle am 8.3.1991 ergibt eine deutliche Verbesserung der Erinnerungsleistungsfähigkeit, Patient kann das Datum wieder exakt angeben, verarbeitet Nachrichten aus dem Fernsehen in richtiger Weise und speichert sie im Gedächtnis einigermaßen normal. Der GDS-Wert fällt von 5 auf 3, der MMS-Wert steigt von 12 auf 25.
- Fall 5:: Patient, 57 Jahre, weiblich, Erstuntersuchung am 20.1.1992. Es findet sich eine relativ stark eingeschränkte Hirnleistungsfunktion. Das Erkennen und Bezeichnen von Gegenständen gelingt nicht, Wiederholungen von Worten oder kurzen Sätzen ist unmöglich, einfache 1x1-Aufgaben können nicht gelöst werden, Lesen ist ebenfalls nicht möglich, die Patientin ist zeitlich und räumlich nicht orientiert, neurologisch und hirnorganisch finden sich keine Besonderheiten. GDS-Score ist 6, der MMS-Wert beträgt 4.
Diagnose: Morbus Alzheimer

Therapie: NADPH 2 mg intravenös, 2x die Woche, Kontrolle am 17.2.1992, die kognitiven Funktionen sind deutlich gebessert, einfache Rechenaufgaben können gelöst werden, einfache Schlußfolgerungen gezogen werden, die zeitliche und räumliche Orientiertheit hat sich signifikant gebessert, der GDS-Wert ist auf 4 abgesunken, der MMS-Wert auf 16 angestiegen. Die 4-wöchige Therapie wurde jeweils im Abstand von einem Monat wiederholt, Verbesserung des Zustandes ist gleich geblieben, Nebenwirkungen wurden nicht beobachtet, Patientin fühlte sich psychisch und physisch wesentlich wohler unter der Therapie.

Bei der erfindungsgemäßen Verwendung von NADH, NADPH oder deren physiologisch verträglichen Salzen können diese in üblicher Weise mit pharmazeutisch annehmbaren Füllstoffen konfektioniert werden bzw. zur Verwendung als Arzneimittel in übliche galenische Zubereitungsformen für die orale, parenterale sowie nasale Anwendung eingearbeitet werden. Die Präparate können in fester Form als Tabletten, Kapseln oder Dragees, in flüssiger Form als Lösungen, Suspensionen, Sprays und Emulsionen sowie Zubereitungsformen mit verzögerter Wirkstoffabgabe vorliegen.

Die in der vorstehenden Beschreibung sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung der reduzierten Form von Nikotinamid-adenin-dinukleotid (NADH), der reduzierten Form von Nikotinamid-adenin-dinukleotidphosphat (NADPH) oder eines physiologisch verträglichen Salzes derselben zur Herstellung eines Arzneimittels zur Behandlung von Morbus Alzheimer, wobei die gemeinschaftliche Verwendung von NADH bzw. NADPH mit monosubstituierten selenorganischen Verbindungen ausgenommen wird.

2. Verwendung gemäß Anspruch 1, wobei NADH oder ein physiologisch verträgliches Salz davon in einer Einzeldosis von 1 mg bis 50 mg bereitgestellt wird.

3. Verwendung gemäß Anspruch 2, wobei die Einzeldosis 1 mg bis 25 mg beträgt.

4. Verwendung gemäß Anspruch 1, wobei NADPH oder ein physiologisch verträgliches Salz davon in einer Einzeldosis von 1 mg bis 20 mg bereitgestellt wird.

## Claims

1. Use of the reduced form of nicotinamide adenine dinucleotide (NADH), the reduced form of nicotinamide adenine dinucleotide phosphate (NADPH), or a physiologically compatible salt thereof for the preparation of a medicament for the treatment of Alzheimer's disease wherein the common use of NADH or NADPH with mono-substituted selenorganic compounds is disclaimed.

2. Use according to claim 1 wherein NADH or a physiologically compatible salt thereof is provided in an individual dose of from 1 mg to 50 mg.

3. Use according to claim 2, wherein the individual dose is 1 mg to 25 mg.

4. Use according to claim 1 wherein NADPH or a physiologically compatible salt thereof is provided in an individual dose of 1 mg to 20 mg.

## Revendications

1. Utilisation de la forme réduite du nicotinamide-adénine-dinucléotide (NADH), de la forme réduite du nicotinamide-adénine-dinucléotide-phosphate (NADPH) ou d'un sel physiologiquement toléré de ces substances pour la préparation d'un médicament destiné au traitement de la maladie de Alzheimer, l'utilisation concomitante de NADH ou NADPH avec des composés séléno-organiques monosubstitués étant exclue.

2. Utilisation selon la revendication 1, NADH ou un sel physiologiquement toléré de ce dernier étant préparé en une dose individuelle de 1 mg à 50 mg.

3. Utilisation selon la revendication 2, la dose individuelle étant de 1 mg à 25 mg.

4. Utilisation selon la revendication 1, NADPH ou un sel physiologiquement toléré de ce dernier étant préparé en une dose individuelle de 1 mg à 20 mg.
